# EUROPÄISCHE PATENTANMELDUNG

(11) **EP 1 043 394 A2**
(43) Veröffentlichungstag der Anmeldung: **11.10.2000**
(21) Anmeldenummer: 00107649.6
(22) Anmeldetag: 08.04.2000
(51) Int. Cl.: C12N 9/64, G01N 33/96, C07K 16/40

(54) **Prostata-spezifisches-Antigen (PSA) enthaltende Zusammensetzung**

(30) Priorität: 10.04.1999 DE 19916242
(71) Anmelder: SERATEC Gesellschaft für Biotechnologie mbH, 37079 Göttingen (DE)
(72) Erfinder: Volk, Matthias, Dr., 37079 Göttingen (DE); Mast, Wolf-Peter, Dr., 37079 Göttingen (DE)
(74) Vertreter: Patentanwälte Rehberg + Hüppe

(57) **Zusammenfassung**

Eine Substanz, deren Gesamtkonzentration an Prostata-spezifischem Antigen (PSA) mit einer N-terminalen Aminosäuresequenz der Folge IVGGWECEKHSQPWQVLVAS mindestens 90 Gewichts-% beträgt, weist mindestens eine Isoform des PSA, deren isoelektrischer Punkt um mindestens 0,6 pI unter dem isoelektrischen Punkt von nicht-glykosiliertem PSA liegt, in einem Anteil von mindestens 3,0 % der PSA-Gesamtkonzentration auf. Die Substanz ist beispielsweise als Referenzsubstanz bei der Bestimmung der Konzentration von PSA in menschlichem Blut oder Serum oder bei der Erzeugung von monoklonalen isoformspezifischen PSA-Antikörpern vorteilhaft verwendbar.

## Beschreibung

Die Erfindung bezieht sich auf eine PSA-haltige Substanz und auf spezielle Verwendungen dieser Substanz. Genauer geht es um eine Substanz, deren Gehalt an Prostata-spezifischem Antigen (PSA), das eine N-terminale Aminosäuresequenz der Folge IVGGWECEKHSQPWQVLVAS aufweist, auf mindestens 90 Gewichts-% aufkonzentriert und aufgereinigt wurde, und deren Verwendungen.

Von Seiten der Industrie, der Universitäten und aus dem Krankenhausbereich wird schon seit längerem gefordert, die Standardisierung des Tumormarkers PSA zu verbessern. Diese Forderung beruht auf der Tatsache, daß PSA ein weltweit akzeptierter Marker für die Diagnose von Prostatakrebs ist und alle diagnostische Wertigkeit auf der genauen Quantifizierung dieses Proteins im Blut oder Serum beruht.

Bei PSA handelt es sich um ein Glycoprotein mit einer anscheinenden Masse von 33 kDa und einer hohen Heterogenität in der glykosidischen Struktur. PSA ist eine Serin-Protease. Seine natürliche Aufgabe besteht in der Verflüssigung des Seminalplasmas. Zudem ist PSA eines der drei meist-exkretierten Proteine der Prostata. Aus Watt et al. in Proc. Natl. Acad. Sci. 83: 3166-3170, 1986 ist es bekannt, daß natives PSA aus Seminalplasma in mindestens fünf Isoformen mit wesentlichem Anteil an der PSA-Gesamtkonzentration in Seminalflüssigkeit vorkommt, die sich durch den Grad ihrer Glycolisierung unterscheiden. Aus dieser Veröffentlichung ist auch die N-terminale Aminosäuresequenz der Folge IVGGWECEKHSQPWQVLVAS für PSA bekannt.

Normalerweise gelangt PSA nicht in signifikanten Mengen in den Blutstrom. Vornehmlich in Fällen von Prostata-Tumoren oder bei benigner Prostatahyperplasie gelangen höhere Konzentrationen ins Blut, bedingt durch eine Schwächung der Blut-Gewebeschranke.

Im Blut wird PSA in wechselndem Umfang von anderen Plasmaproteinen komplexiert (z.B. Antichymotrypsininhibitor, ACT oder α2 Makroglobulin, A2M). Generell sind Komplexe von PSA mit A2M nicht detektierbar wohingegen Addukte mit ACT als sogenanntes "komplexiertes PSA" Eingang in die Literatur fanden.

Die Messung von prostataspezifischem Antigen (PSA) im Serum gilt heutzutage als die Methode der Wahl, um Prostatatumoren nichtinvasiv diagnostizieren zu können. Zudem werden PSA-Messungen bei der Tumor-Therapiekontrolle durchgeführt. Somit sind PSA-Messungen im Serum oder Vollblut wichtig für das präoperative Screening und das postoperative Monitoring von Prostatatumoren. Da in allen Fällen die klinische Bedeutung von PSA auf quantitativen Messungen beruht, ist ein international akzeptierter Standard mit genau bekannter Zusammensetzung und Herkunft von entscheidender Bedeutung.

Aus Jurinic-Winkler, C. et al. in Eur. Urol. 24: 487-491, 1993 und Malm, J. und Lilja, H. in Scand. J. Clin. Lab. Invest. 55 Suppl. 221: 15-22, 1995 ist es bekannt, daß bei Patienten mit PSA-Serumkonzentrationen von 4 ng/mL bis zu 10 ng/mL zu ca. 45% eine benigne Prostatahyperplasie und zu ca. 22-46% ein Prostatatumor vorliegt. Zudem zeigten 59% bis 71% von Patienten mit Prostatatumor eine PSA Konzentration von mehr als 10 mg/mL Serum. PSA-Konzentrationen über 4 ng/mL Serum wurden altersbedingt gefunden in 2% der Altersgruppe 50-59 Jahre, in 13% der Altersgruppe 60-69 Jahre und in 19% der Altersgruppe 70-79 Jahre. Wichtig ist jedoch auch, daß trotz normaler Serum-PSA Konzentration in einigen Fällen dennoch ein Prostatatumor vorliegen kann. Eine sehr umfangreiche Studie wird momentan von der Europäischen Gemeinschaft durchgeführt (European Randomized Study of Screening for Prostate Cancer; teilweise veröffentlicht durch Bangma, C. H. et al. in Urologic Clinics of North America, 24, No. 2:307-314, 1997).

Eine primäre Referenzsubstanz für die Bestimmung der Konzentration von PSA in menschlichem Blut oder Serum mit einer Konzentration von mindestens 90 Gewichts-% an PSA sollte nur aus freiem PSA bestehen und sollte in seiner Zusammensetzung von nativem Material möglichst nicht abweichen und auch möglichst alle natürlich vorkommenden Isoformen beinhalten, um Abweichungen bei der Kalibrierung zu vermeiden: es besteht durchaus die Möglichkeit, daß Isoformen mit unterschiedlichen Kohlenhydrat-Ketten unterschiedliche Affinitäten zu A2M und ACT aufweisen. Zudem besteht die Hypothese, daß maligne und benigne Prostatazellen voneinander abweichende Isoformgemische exkretieren können. Der bekanntermaßen differierende Grad der Komplexierung könnte somit seine Ursache haben in den differierenden Exkretionsmustern maligner und benigner Zellen. Die Verwendung von komplexiertem PSA, wie es im Blut oder Serum vorkommt, ist für einen primären Standard nicht ratsam, da sowohl die Bedeutung von komplexiertem PSA als auch seine Stabiliät und Konzentration starken Schwankungen unterliegt.

Ein Verfahren zum Aufreinigen von PSA aus Seminalflüssigkeit ist aus Sensabaugh, G. F. und Blake, E. T. in J. Urol 144: 1523-1526, 1990 bekannt. Dieses Verfahren macht von einem Kationentauscher Gebrauch. Das bekannte Verfahren führt zu einer signifikanten Artefaktbildung bedingt durch Autoproteolyse, da es im wesentlichen in dem pH-Bereich durchgeführt wird, in dem PSA als auch gegen sich selbst gerichtete Protease wirksam ist. So ist die erreichbare Ausbeute an nativem PSA nur klein ebenso wie die Konzentration an nativem PSA in einer aufgereinigten Substanz. Zudem ist die Ausbeute verschieden-glykosilierter Isoformen System-bedingt unterschiedlich. So wurden von Belanger, A. et al. in The prostate 27: 187-197, 1995 in einer nach Sensabaugh und Blake aufgereinigten PSA-haltigen Substanz nur zwei Isoformen mit nennenswerter Konzentration gefunden, obwohl mindestens fünf Isoformen bekannt sind.

Der Erfindung liegt die Aufgabe zugrunde, eine Substanz aufzuzeigen, deren Gehalt an Prostata-spezifischem Antigen (PSA), das eine N-terminale Aminosäuresequenz der Folge IVGGWECEKHSQPWQVLVAS aufweist, über 90 Gewichts-% liegt und bei der die nachweisbaren Isoformen des PSA ein Beleg für eine Artefakt-freie Aufreinigung sind.

Diese Aufgabe wird durch eine Substanz gelöst, deren Gesamtkonzentration an Prostata-spezifischem Antigen (PSA) mit einer N-terminalen Aminosäuresequenz der Folge IVGGWECEKHSQPWQVLVAS mindestens 90 Gewichts-% beträgt, wobei mindestens eine Isoform des PSA, deren isoelektrischer Punkt um mindestens 0,6 pI unter dem isoelektrischen Punkt von nicht-glykosiliertem PSA liegt, in einem Anteil von mindestens 3,0 % der PSA-Gesamtkonzentration vorliegt.

An der erfindungsgemäßen Substanz ist bereits als neu anzusehen, daß Sie tatsächlich eine Gesamtkonzentration an Prostata-spezifischem Antigen mit der bekannten N-terminalen Aminosäuresequenz von mindestens 90 Gewichts-% aufweist. Nach Sensabaugh und Blake aufgereinigte PSA-haltige Substanzen weisen durch die Autoproteolyse zumindest kein natives PSA in dieser Konzentration auf. Weiterhin ist neu, daß mindestens eine Isoform des PSA, deren isoelektrischer Punkt um mindestens 0,6 pI unter dem isoelektrischen Punkt von nicht-glykosiliertem PSA liegt, in einem signifikanten Anteil der PSA-Gesamtkonzentration existent ist. Diese Existenz ist ein Nachweis dafür, daß auch die bekannten stärker glykosilierten Isoformen des PSA durch die Aufreinigung aufkonzentriert und nicht diskriminiert wurden.

In der bevorzugten Ausführungsform der erfindungsgemäßen Substanz ist mindestens eine Isoform des PSA, deren isoelektrischer Punkt um mehr als 1,0 pI unter dem isoelektrischen Punkt von nicht-glykosiliertem PSA liegt, in einem Anteil von mindestens 3,0 % der PSA-Gesamtkonzentration vorhanden. Hiermit ist auch die Existenz der am stärksten glykosilierten bekannten Isoform des PSA in der Substanz abgestellt, die in einem relevanten Anteil in natürlichen Isoformgemischen von PSA vorkommt.

Insgesamt liegen bei der erfindungsgemäßen Substanz in der Regel mindestens vier Isoformen des PSA, die unterschiedliche isoelektrische Punkte aufweisen, in einem Anteil von jeweils mindestens 3,0 % der PSA-Gesamtkonzentration vor. In der bevorzugten Ausführungsform der Erfindung sind es fünf solcher Isoformen. Dabei weist eine erste Isoform des PSA einen isoelektrischen Punkt von 5,6 bis 5,8 auf und liegt in einem Anteil von 3 bis 5 % der PSA-Gesamtkonzentration vor, während eine zweite Isoform des PSA einen isoelektrischen Punkt von 6,1 bis 6,4 aufweist und in einem Anteil von 9 bis 13 % der PSA-Gesamtkonzentration vorliegt, eine dritte Isoform des PSA einen isoelektrischen Punkt von 6,6 bis 6,8 aufweist und in einem Anteil von 34 bis 42 % der PSA-Gesamtkonzentration vorliegt, eine vierte Isoform des PSA einen isoelektrischen Punkt von 6,9 bis 7,1 aufweist und in einem Anteil von 25 bis 31 % der PSA-Gesamtkonzentration vorliegt und eine fünfte Isoform des PSA einen isoelektrischen Punkt von 7,1 bis 7,3 aufweist und in einem Anteil von 10 bis 14 % der PSA Gesamtkonzentration vorliegt. Der isoelektrische Punkt dieser fünften Isoform entspricht dem berechneten isoelektrischen Punkt für nicht-glykosiliertes PSA.

Die PSA-Gesamtkonzentration der erfindungsgemäßen Substanz beträgt in der Regel nicht nur 90, sondern mindesten 95 Gewicht-%, vorzugsweise sogar mindestens 98 Gewichts-%.

Die molekularen Massen der Isoformen des PSA in der erfindungsgemäßen Substanz, die einen Anteil von jeweils mindestens 3 % der PSA-Gesamtkonzentration aufweisen, liegen typischerweise zwischen 26000 und 32000 DA. Dabei entspricht die molekulare Masse von 26000 DA derjenigen von nicht-glykosiliertem PSA. Oberhalb von 32000 DA treten nur noch vereinzelt sehr stark glykosilierte Isoformen des PSA auf, die aber jeweils keinen relevanten Anteil an der PSA-Gesamtkonzentration mehr ausmachen. In diesem Zusammenhang ist zu betonen, daß die vorstehend angegebenen PSA-Gesamtkonzentrationen an der Substanz sich immer auf PSA mit einer molekularen Masse von mindestens 26000, also auf natives PSA beziehen und es hier nicht um irgendwelche Fragmente von proteolysiertem PSA geht.

Mindestens ein signifikanter Anteil von 3,0 %, vorzugsweise aber von mindestens 10 % der PSA-Gesamtkonzentration in der erfindungsgemäßen Substanz ist phosphoriliert, wobei der phosphorilierte Anteil der PSA-Gesamtkonzentration bestimmt ist, d. h. der phosphorilierte Anteil der PSA-Gesamtkonzentration muß nicht immer denselben Wert annehmen. Es ist aber Merkmal dieser bevorzugten Ausführungsform, daß der jeweilige Wert zahlenmäßig bekannt ist.

In einer besonders bevorzugten Ausführungsform der erfindungsgemäßen Substanz ist mindestens eine Isoform des PSA, deren isoelektrischer Punkt um mindestens 0,4 pI unter dem isoelektrischen Punkt von nicht-glykosiliertem PSA liegt und die den Anteil von mindestens 3,0 % der PSA-Gesamtkonzentration aufweist, zumindest 50 % phosphoriliert. Es ist davon auszugehen, daß phosphoriliertes PSA im Serum oder Blut im Vergleich zu nicht-phosphoriliertem PSA weniger stark oder gar nicht zur Komplexbildung neigt. Entsprechend ist die erfindungsgemäße Substanz von besonderem Interesse, wenn sie von dem nicht zur Komplexbildung neigenden phosphoriliertem PSA hohe Anteile enthält. Bei der Aufreinigung von natürlich vorkommendem PSA zu der erfindungsgemäßen Substanz hat sich gezeigt, daß die phosphorilierten Isoformen zumindest einen gewissen Glykosilierungsgrad aufweisen, der durch den Mindestabstand von 0,4 pI zu nicht-glykosiliertem PSA beim isoelektrischen Punkt charakterisiert ist.

Die erfindungsgemäße Substanz erfährt eine besondere erfindungsgemäße Verwendung als Referenzsubstanz bei der Bestimmung der Konzentration von PSA im menschlichen Blut oder Serum. Gerade hier ist der definierte Anteil an phosphoriliertem PSA im Bereich der stärker glykosilierten Isoformen des PSA bei der Substanz von besonderem Interesse.

Eine weitere Verwendung der erfindungsgemäßen Substanz ist bei der Erzeugung von monoklonalen Isoform-spezifischen PSA-Antikörpern gegeben. Die Bildung von Antikörpern gegen PSA kann in bekannter Weise durch Injektion der erfindungsgemäßen Substanz in den Organismus, beispielsweise von Mäusen provoziert werden. Die erzeugten Antikörper können durch Austestung dahingehend sortiert werden, ob sie isoformspezifisch gegen eine in der Substanz enthaltene Isoform des PSA reagieren.

Die Erfindung wird im folgenden durch Herstellungsbeispiele, Verwendungsbeispiele und Versuchsergebnisse zu der erfindungsgemäßen Substanz näher erläutert und beschrieben; dabei zeigt:
- Fig. 1: SDS-PAGE Analysen von PSA, das mit oder ohne die Zugabe von Serin-Proteaseinhibitor aufgereinigt wurde;
- Fig. 2: eine isoelektrische Fokussierung (IEF) von gereinigtem PSA;
- Fig. 3: eine Überprüfung der Reinheit von gereinigtem PSA mittels RP18 HPLC;
- Fig. 4: eine N-terminale Sequenzanalyse von PSA;
- Fig. 5: eine hochauflösende HPLC von gereinigtem PSA;
- Fig. 6: ein MALDI-Massenspektrogramm von PSA;
- Fig. 7: ein Elektrospray-Massenspektrogramm von PSA;
- Fig. 8: einen Nachweis phosphorilierter Aminosäuren bei PSA und
- Fig. 9: eine Immunologische Reaktion von anti-PSA-Antikörpern mit PSA-Isomeren auf IEF-Gel Westernblots.

### 1. Aufreinigung und Charakterisierung von PSA

### 1. 1. Ursprung des Materials

PSA wurde aus 2 L menschlicher Seminalflüssigkeit aufgereinigt. Jeder Spender wurde negativ auf HIV I/II, HCV und Hepatitis B Infektion hin überprüft. Die Proben aller getesteten Spender wurden vereinigt und in Aliquots von jeweils 50 mL bei -80°C gelagert.

### 1. 2. Aufreinigung von PSA aus humaner Seminalflüssigkeit

Im folgenden werden drei verschiedene Methoden der Aufreinigung beschrieben, die eine Gewinnung von PSA mit guten Ausbeuten und vernachlässigbarer Artefaktbildung ermöglichten.

### 1.3. METHODE 1

### A. Zentrifugation und Dialyse

Seminalflüssigkeit wurde bei +4°C über Nacht aufgetaut und niedertourig zentrifugiert (20 min bei 4200 g). Der Überstand, das Seminalplasma, wurde für die Aufreinigung von PSA und anderer Tumormarkerproteine verwendet (z.B. prostataspezifisches Protein, PSP94 und saure Phospatase, PAP). Das Zentrifugat (Spermatozoen) wurde mit flüssigem Stickstoff eingefroren und anderen Verwendungen zugeführt.

Der Überstand der Zentrifugation wurde dialysiert gegen eine Membran mit einer Porengröße durchlässig für Proteine > 20 kDa. Als Puffer wurde 20 mmol/L Na-Phosphat pH = 6,8 verwendet. Durch diesen initialen Schritt wurden aus dem Seminalplasma alle Moleküle mit einer Masse < 20 kDa entfernt, jedoch PSA mit einer anscheinenden Masse von 33 kDa zurückgehalten.

Anschließend wurde gegen 20 mmol/L Na-Phosphat pH = 6,8 dialysiert mit einer Membran einer Porengröße von 50 kDa. Das Dialysat wurde bei +4°C mit Ammoniumsulfat gefällt (die Endkonzentration an (NH₄)₂SO₄ betrug 472 g/L). Das so aufkonzentrierte Material wurde gegen 50 mmol/L Tris-HCl pH = 8,2 unter Verwendung einer Membran mit einer Porengröße von 20 kDa erneut dialysiert. Es fanden drei Pufferwechsel statt.

### B) Affinitätschromatographie

Die monoklonalen Antikörper PSA 28 A4 und PSA 43 A2, die auch im SERATEC PSA ELISA der Anmelderin verwendet werden, wurden unter der Anwendung üblicher Verfahren an Tresylsepharose gekoppelt. Diese beiden Antikörper binden alle Isoformen von PSA und zudem PSA-ACT-Komplexe. Die Spezifikationen der verwendeten Antikörper sind im folgenden beschrieben:

### Erster Antikörper:

- KLON:: PSA 28 / A4
- SPEZIFITÄT:: Humanes prostataspezifisches Antigen
- QUELLE:: Murine Aszites
- REINIGUNG:: Ammoniumsulfat-Fällung gefolgt von Protein G - und Anionenaustauschchromatographie
- VIRUSINAKTIVIERUNG:: pH 2,5 5 min
- KLASSE UND SUBKLASSE:: IgG₁ , Kappa
- AFFINITÄT:: K_{d} = 10⁻⁸ Mol/L

### Zweiter Antikörper:

- KLON:: PSA 43 A 2
- SPEZIFITÄT:: Humanes prostataspezifisches Antigen
- QUELLE:: Murine Aszites
- REINIGUNG:: Ammoniumsulfat-Fällung gefolgt von Protein G - und Anionenaustauschchromatographie
- VIRUSINAKTIVIERUNG:: pH 2,5 5 min
- KLASSE UND SUBKLASSE:: IgG₂ₐ , Kappa
- AFFINITÄT:: K_{d} = 10⁻¹⁰ Mol/L

Die Effizienz der Kopplung betrug für beide Antikörper ca. 75 bis 100 mg/mL Sepharose. Dies wurde über die Bestimmung der Proteinkonzentration im Überstand vor und nach der Kopplung bewiesen.

Zum Zwecke der Gewinnung von PSA aus Seminalflüssigkeit wurde das fraktioniert-dialysierte Material auf die antikörperbeschichtete Tresylsepharose aufgetragen und mittels eines Guanidinium-HCl -Gradienten eluiert. Die experimentellen Bedingungen waren wie folgt:
- Säulendimensionen:: 1 x 5 cm
- Probengröße pro Zyklus:: 10 ml dialysiertes Seminalplasma
- Flußrate:: 1 mL min⁻¹
- Detektionswellenlänge:: 280 nm
- Verwendeter Gradient:: *Eluent A*: 10 mmol/L Na-Phosphat pH = 8.2
*Eluent B*: Eluent A + 1 mol/L Guanidinium * HCl; pH = 8.2

| Zeit / min | Volumenfraktion B / % |
|---|---|
| 0 | 0 |
| 10 | 0 |
| 100 | 70 |
| 120 | 100 |
| 150 | 100 |

PSA eluierte ab ca. 60% Eluent B. Unverzüglich nach der Elution wurden PSA-haltige Fraktionen gegen Eluent A dialysiert mit einmaligem Pufferwechsel nach 12 h.

### C. RP-C18 HPLC

Das so gewonnene Material wurde mittels HPLC an Umkehrphasen C18-Säulen endgereinigt. Hierbei wurde eine LiChrosorp 250-4, 5µm, Säule von Merck®, Darmstadt unter folgenden Bedingungen eingesetzt:
- Protein pro Zyklus:: max. 3 mg
- Durchflußrate:: 0.5 mL min⁻¹
- Detektionswellenlänge:: 290 nm
- Verwendeter Gradient:: Eluent A: 0.1 % (v/v) Trifluoressigsäure (TFA) in H₂O
Eluent B: Acetonitril (> 99.9 % v/v) + 0.1% (v/v) TFA

| Zeit / min | Volumenfraktion B / % |
|---|---|
| 0 | 0 |
| 35 | 0 |
| 90 | 55 |
| 95 | 100 |
| 100 | 0 |

Alle PSA-haltigen Eluate wurden vereinigt, mit flüssigem Sticktoff eingefroren und gefriergetrocknet. Das so erhaltene salz- und detergentienfreie PSA wurde bis zur weiteren Verwendung bei -70°C gelagert.

### 1.4. METHODE 2

Seminalflüssigkeit wurde bei +4°C über Nacht aufgetaut und niedertourig zentrifugiert (20 min bei 4200 g). Der Überstand, das Seminalplasma, wurde für die Aufreinigung von PSA und anderer Tumormarkerproteine (z.B. prostataspezifisches Protein, PSP94 und saure Phospatase, PAP) verwendet. Das Zentrifugat (Spermatozoen) wurde mit flüssigem Stickstoff eingefroren und anderen Verwendungen zugeführt.

Das erhaltene Seminalplasma wurde mit dem gleichen Volumen von 10 mmol/L Tris-HCl pH = 8,2 versetzt und mit Ammoniumsulfat bei +4°C gefällt. Die Endkonzentration betrug 472 g/L. Nach 6 h Lagerung bei +4°C wurde niedertourig zentrifugiert und anschließend gegen 10 mmol/L Tris-HCl pH = 8,2 dialysiert (Porengröße <10 kDa). Es fand ein dreimaliger Pufferwechsel in regelmäßigen Abständen statt.

### A. Anionenaustauschchromatographie

Das dialysierte Material wurde anschließend nochmals zentrifugiert und der Überstand für eine Anionenaustauschchromatographie verwendet. Hierbei wurde Diethylaminoethyl-(DEAE)-Fraktogel (Merck®, Darmstadt) verwendet. Die chromatographischen Bedingungen waren wie folgt:
- Säulendimensionen:: 2 x 25 cm
- Probenvolumen:: 50 mL dialysiertes Seminalplasma
- Flußrate:: 1 mL min⁻¹
- Detektionswellenlänge:: 300 nm
- Gradient :: *Eluent A*: 10 mmol/L Tris-Cl pH = 8.2
*Eluent B*: Eluent A + 0.5 mol/L NaCl

| Zeit / min | Volumenfraktion B / % |
|---|---|
| 0 | 0 |
| 110 | 0 |
| 330 | 20 |
| 380 | 100 |
| 400 | 100 |

PSA eluierte zwischen 5 % (v/v) und 20 % (v/v) Eluent B (25 - 100 mmol/L NaCl). Dies wurde bewiesen durch Polyacrylamid-Gelelektrophorese in der Gegenwart von Na-Dodezylsulfat (SDS-PAGE) und mittels ELISA-Messungen.

Fraktionen, die in signifikanter Menge PSA enthielten wurden vereinigt und bei +4°C mit (NH₄)₂SO₄ bei einer Endkonzentration von 472 g/L gefällt. Das gefällte Material wurde anschließend für 48 h gegen 50 mmol/L Tris-HCl pH = 8.2 dialysiert. Hierbei wurde der Puffer dreimal in regelmäßigen Abständen gewechselt.

### B. Gelfiltration

Mit dem dialysierten Material wurde anschließend eine Gelfiltration bei +4°C durchgeführt. Hierbei wurde Sephacryl S200 (Pharmacia®, Uppsala, Sweden) verwendet.

Die Säulendimensionen betrugen 3.5 x 70 cm. Als Eluent wurde 50 mmol/L Tris-HCl pH = 8.2 + 100 mmol/L NaCl bei einer Flußrate von 0.5 mL min⁻¹ verwendet. Für jeden Lauf wurden maximal 4,5 mL PSA-haltiger Lösung auf die Säule aufgetragen. Alle PSA-haltigen Fraktionen wurden vereinigt und bei +4°C bis zur weiteren Verwendung gelagert.

### C. RP-C18 HPLC

Das so gewonnene Material wurde mittels HPLC an Umkehrphasen C18-Säulen endgereinigt . Hierbei wurde eine LiChrosorp 250-4, 5µm, Säule von Merck®, Darmstadt unter folgenden Bedingungen eingesetzt:
- Protein pro Zyklus:: max. 3 mg
- Durchflußrate:: 0.5 mL min⁻¹
- Detektionswellenlänge:: 290 nm
- Verwendeter Gradient:: Eluent A: 0.1 % (v/v) Trifluoressigsäure (TFA) in H₂O
Eluent B: Acetonitril (> 99.9 % v/v) + 0.1% (v/v) TFA

| Zeit / min | Volumenfraktion B / % |
|---|---|
| 0 | 0 |
| 35 | 0 |
| 90 | 55 |
| 95 | 100 |
| 100 | 0 |

Alle PSA-haltigen Eluate wurden vereinigt, mit flüssigem Stickstoff eingefroren und gefriergetrocknet. Das so erhaltene salz- und detergentienfreie PSA wurde bis zur weiteren Verwendung bei -70°C gelagert.

### 1.5. Methode III

Seminalflüssigkeit wurde bei +4°C über Nacht aufgetaut und niedertourig zentrifugiert (20 min bei 4200 g). Der Überstand, das Seminalplasma, wurde für die Auf reinigung von PSA und anderer Tumormarkerproteine verwendet (z.B. prostataspezifisches Protein, PSP94 und saure Phospatase, PAP). Das Zentrifugat (Spermatozoen) wurde mit flüssigem Stickstoff eingefroren und anderen Verwendungen zugeführt.

Das erhaltene Seminalplasma wurde mit dem gleichen Volumen von 10 mmol/L Tris-HCl pH = 8,2 versetzt und mit Ammoniumsulfat bei +4°C gefällt. Die Endkonzentration betrug 472 g/L. Nach 6 h Lagerung bei +4°C wurde niedertourig zentrifugiert und anschließend gegen den folgenden Puffer dialysiert:
20 mmol/L Tris-HCl pH = 7.4
0.5 mmol/L NaCl
1mmol/L CaCl₂
1 mmol/ L MnCl₂

### A) Lektin-Affinitätschromatographie über ConA-Sepharose

Die ConA-Sepharose 4B wurde mit dem oben spezifizierten Puffer equilibriert. Es wurden 25 mL dieser Suspension verwendet, um eine Säule der Abmessung 1,5 x 20 cm zu befüllen. Auf diese Säule wurden 40 mL Seminalplasma aufgetragen und der Durchlauf noch zweimal über die Säule geleitet.

Anschließend wurde die Säule solange mit ConA-Puffer gespült, bis kein Material mit einer Eigenabsorption bei 280 nm mehr eluierte. Anschließend wurden gebundene Glykoproteine mittels 250 mmol/L Methyl-α-D-Mannopyranosid in Con-A-Puffer eluiert. Das Eluat wurde gegen 5 L 20 mmol /L Tris-HCl pH = 8,0 dialysiert. Die so erhaltene Lösung (120 mL) wurde mittels Ultrafiltration auf ein Endvolumen von 11 mL aufkonzentriert.

### B) Anionenaustauschchromatographie mittels Mono-Q-FPLC

Das erhaltene Konzentrat (11 L) wurde auf eine Mono-Q-Säule aufgetragen. Die experimentellen Bedingungen waren wie folgt:
- Säulendimensionen:: 3 x 15 cm
- Volumen pro Lauf:: 10 mL Con-A-gebundenes Material
- Flußrate:: 1 mL min⁻¹
- Detektionswellenlänge:: 280 nm
- Gradient:: *Eluent A*: 20 mmol/L Tris-HCl pH = 8.0
*Eluent B*: Eluent A + 1 mol/L NaCl

| Zeit / min | Volumenfraktion B / % |
|---|---|
| 0 | 0 |
| 30 | 0 |
| 100 | 20 |
| 120 | 100 |
| 150 | 100 |

PSA eluierte etwa zwischen 68 und 80 min. Dieses PSA-haltige Material wurde vereinigt und mittels Festphasenextraktion über C18-Kartuschen entsalzt.

### C) Festphasenextraktion

Hierbei wurden pro Lauf maximal 5 mL des Mono-Q-Eluates aufgetragen und mit 20 mL H₂O + 0.1 % (v/v) TFA gespült. Eluiert wurde mit 10 mL 60% (v/v) Acetonitil in H₂0 + 0.1% TFA.

Das hierdurch entsalzte PSA wurde mit flüssigem Sticktoff eingefroren, anschließend gefriergetrocknet und bis zur weiteren Verwendung bei -70 °C gelagert.

### 2. Ergebnisse

### 2. 1. Aufreinigungstabellen

PSA wurde aus humaner Seminalflüssigkeit mit einer Ausbeute von 20 - 50 % je nach angewandter Methode gewonnen. Hierbei wurden bei der Methode I fraktionierte Dialyse, Affinitätschromatographie und HPLC über RP18-Säulen angewandt. Methode II beinhaltete Anionenaustauschchromatographie, Gelfiltration und HPLC über RP18-Säulen. Bei Methode III wurden ConcavalinA-(Lektin)-Affinitätschromatographie, Anionenaustauschchromatographie und Festphasenextraktion verwendet.

Die Aufreinigungstabellen für alle drei angewandten Methoden sind in den folgenden Tabellen 1 bis 3 aufgeführt.

**Tabelle 1**

| **Aufreinigung von PSA aus 10 mL humaner Seminalflüssigkeit nach Methode I** | | | |
|---|---|---|---|
| Fraktion | Volumen / mL | PSA Masse* / mg | PSA verbleibend / % |
| Roh | 10,0 | 10,12 | 100,0 |
| Dialyse | 32,0 | 8,40 | 83,0 |
| Affinitätschromatographie | 8,7 | 7,32 | 72,3 |
| RP₁₈ HPLC | 5,4 | 5,59 | 55,2 |

| | | | |
|---|---|---|---|
| * Die Masse wurde mittels ELISA bestimmt | | | |

**Tabelle 2**

| **Aufreinigung von PSA aus 50 mL humaner Seminalflüssigkeit nach Methode II** | | | |
|---|---|---|---|
| Fraktion | Volumen / mL | PSA Masse* / mg | PSA verbleibend / % |
| Roh | 50,0 | 59,45 | 100,00 |
| (NH₄)₂SO₄ Fällung und Dialyse | 44,0 | 55,20 | 92,85 |
| DEAE | 12,5 | 49,40 | 83,10 |
| S-200 | 87,0 | 38,26 | 64,36 |
| RP₁₈ | 16,0 | 21,18 | 35,63 |

| | | | |
|---|---|---|---|
| * Die Masse wurde mittels ELISA bestimmt | | | |

**Tabelle 3**

| **Aufreinigung von PSA aus 160 mL humaner Seminalflüssigkeit nach Methode III** | | | |
|---|---|---|---|
| Fraktion | Volumen / mL | PSA Masse* / mg | PSA verbleibend / % |
| Roh | 160,0 | 187,50 | 100,0 |
| Con A Sepharose | 120,0 | 84,38 | 45,0 |
| Mono Q | 12,0 | 48,75 | 26,0 |
| Festphasenextraktion | 10,0 | 37,50 | 20,0 |

| | | | |
|---|---|---|---|
| * Die Masse wurde mittels ELISA bestimmt | | | |

Offensichtlich liefert die Methode I bessere Resultate als die Methoden II und III: Die Ausbeute ist hoch und die benötigte Zeit geringer als z.B. bei Methode II. Die Methode III liefert die geringsten Ausbeuten, hat jedoch den Vorteil, daß größere Volumina bearbeitet werden können. Somit scheint die Methode III für eine Massenproduktion am besten geeignet zu sein.

In jedem Fall wurde jedoch gezeigt, daß die im Folgenden aufgeführten Charakteristika des gereinigten PSA keine Unterschiede je nach angewandter Methode aufwiesen.

### 2.2. Beweis der Integrität des gewonnenen PSA

Bei der Analyse von PSA mittels SDS-PAGE stellt man fest, daß in Gegenwart reduzierender Agentien (β-Mercaptoethanol) neben einer Bande bei ca. 33 kDa zwei weitere Banden bei ca. 25 und 10 kDa auftreten. Diese Banden treten bei SDS-PAGE ohne reduzierende Agentien nicht auf. Bei diesen niedermolekulareren Banden handelt es sich um intern-autoproteolytisch gespaltenes PSA mit einer natürlichen Häufigkeit von ca. 5 - 10 % (vergleiche die Druckschrift Watt et al. aus der Beschreibungseinleitung).

Generell kann PSA vor, während oder nach der Exkretion in das Seminalplasma intern-gespalten werden. Eine Spaltung kann jedoch auch erst während der Aufreinigung geschehen. Um zu analysieren, zu welchem Zeitpunkt intern-gespaltenes PSA entsteht, wurde in Parallelversuchen PSA aus humaner Seminalflüssigkeit mit oder ohne Serin-Proteaseinhibitor aufgereinigt.

Es wurden 50 mL Seminalflüssigkeit für Parallelversuche wie folgt verwendet:

Zum Beispiel bei Methode II wurde [4-(2-Aminoethyl)-Benzylsulfonylfluorid x HCl ; Pefabloc, Boehringer®, Mannheim] unmittelbar nach Auftauen zur Hälfte der Probenmenge (25 mL) bis zu einer Endkonzentration von 0.5 mmol/L zugesetzt. Die zweite Hälfte der Probe (25 mL) wurde ohne Pefablock gereinigt.

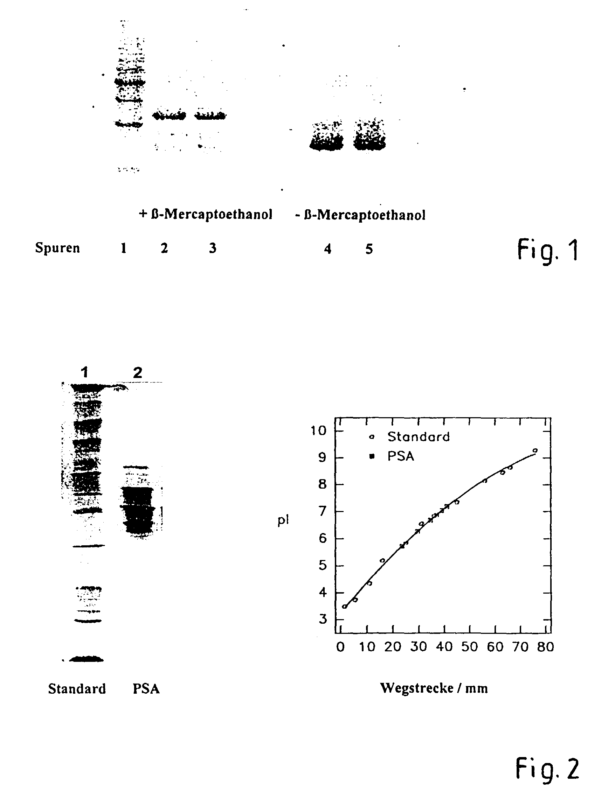

Nach der Durchführung der kompletten Aufreinigungsprozedur wurden von beiden parallel erhaltenen Materialien SDS-Gelelektrophoresen mit und ohne β-Mercaptoethanol durchgeführt. Wie in Fig. 1 ersichtlich, zeigte sich kein Unterschied bei den Präparationen mit und ohne Proteaseinhibitor. Zudem war in beiden Präparationen ohne Reduktionsmittel keine Bande bei 25 bzw. 10 kDa sichtbar.

Den in Fig. 1 dargestellten SDS-PAGE Analysen liegen die folgenden experimentellen Schritte zugrunde: PSA wurde mit Proteaseinhibitor aufgereinigt und mittels SDS-PAGE in Gegenwart von 2 % (v/v) β-Mercaptoethanol (Spur 2) oder ohne β-Mercaptoethanol (Spur 4) analysiert. Der Proteinstandard wurde in Spur 1 aufgetragen. Die Präparation ohne Inhibitor wurde analog durchgeführt, daß heißt (Spur 3) ohne β-Mercaptoethanol, (Spur 5) mit 2 % (v/v) β-Mercaptoethanol. Alle PAGE -Analysen wurden nach Lämmli (1970) mit 0,1 % (w/v) Na-Dodeclysulfat durchgeführt (SDS-PAGE). Hierbei wurden ca. 2 µg Protein pro Spur aufgetragen. Das Trenngel hatte eine Konzentration von 16 % und das Sammelgel von 5 %. Als Standard wurde eine 10 kDa Proteinleiter verwendet (Gibco BRL ®). Proteinfärbungen wurden mit Coomassie Brilliant Blue R-250 ® erreicht.

Die erhaltenen Ergebnisse beweisen, daß PSA während der Aufreinigungsprozedur nicht in signifikanten Mengen zerstört wird. Der Anteil von intern-gespaltenen PSA ist somit offensichtlich integraler Bestandteil des natürlicherweise in der Seminalflüssigkeit vorliegenden Materials.

### 2.3. Charakterisierung des gereinigten PSA

### 2.3.1. Isoelektrische Fokussierung

Eine isoelektrische Fokussierung von gereinigtem PSA ist in Fig. 2 gezeigt, der die folgenden experimentellen Schritte zugrundeliegen: IEF-Analysen wurden auf einem Elektrophoresesystem (Multiphor II, Pharmacia®) durchgeführt, welches mit einer Spannungsquelle ( EPS 3500 XL, Pharmacia®) und einer Kühleinheit (Pfeiffer®) verbunden war. Es wurden vorbeschichtete Polyacrylamid Gele für isoelektrische Fokussierungszwecke verwendet (Ampholine PAGE plate, part no 80-1124-80, Pharmacia®). Die Fokussierung wurde nach den Angaben des Herstellers für den sogenannten breiten pH-Bereich (pH = 3,5 - 9,5) durchgeführt.

Bei der Analyse der Isoformzusammensetzung des gereinigten PSA mittels isoelektrischer Fokussierung (IEF) zeigte sich im Speziellen, daß PSA ein Gemisch von mindestens fünf verschiedenen Isoformen ist. Die isoelektrischen Punkte (pI) lagen bei 5,72; 6,26; 6,68; 7,03 and 7,18. Zudem wurde keine Isoform gefunden, deren pI höher als 7,2 war. Dies kann als Beweis dafür gelten, daß hochreines natives Material erhalten wurde, da der isoelektrische Punkt von nicht-glykosiliertem PSA bei 7,2 liegt und alle natürlicherweise in Glykoproteinen vokommenden Zucker neutral oder sauer reagieren (z.B. Sialinsäure).

Generell wurden Isoformzusammensetzungen mit folgenden Mittelwerten erhalten:

| Isoform | pI | Menge / % |
|---|---|---|
| 1 | 5,72 | 5 |
| 2 | 6,26 | 11 |
| 3 | 6,68 | 38 |
| 4 | 7,03 | 28 |
| 5 | 7,18 | 12 |

### 2.3.2. RP₁₈-HPLC

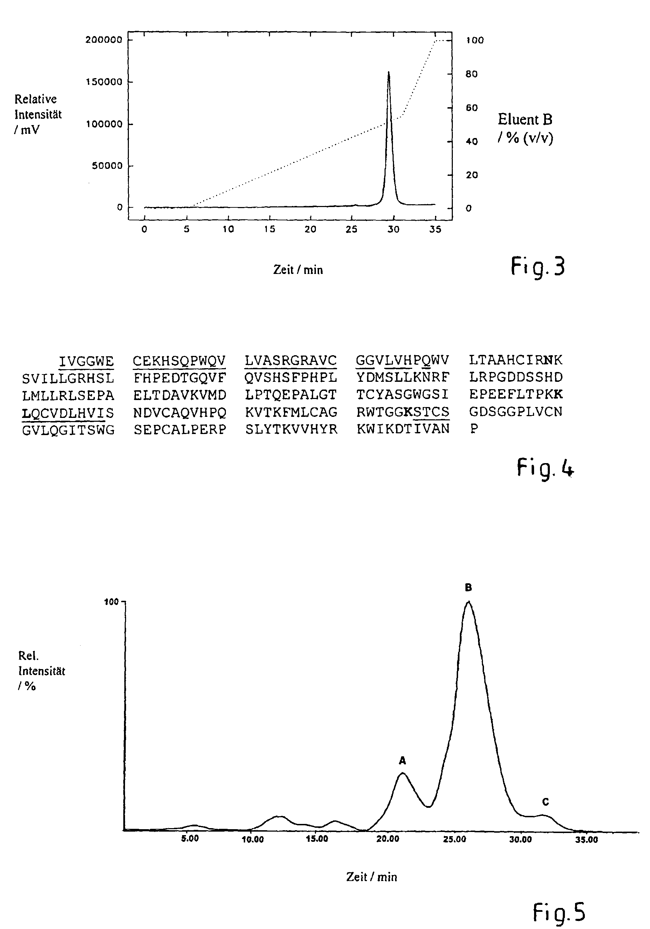

Die Reinheit des aufgereinigten PSA wurde ebenfalls mittels Umkehrphasen-HPLC über C18-Säulen (RP18-HPLC) geprüft. Das Ergebnis ist in Fig. 3 gezeigt. Durch Integration aller sichtbaren Peaks konnte die Reinheit auf > 99% festgelegt werden.

Fig. 3 liegen die folgenden experimentellen Schritte zugrunde: Eine Probe von gereinigtem PSA wurde in Wasser gelöst und auf eine Konzentration von ca. 1 mg/mL eingestellt. Von dieser Lösung wurden 10 µL injiziert. Die Analyse wurde mittels HPLC über C18-Säulen (LiChrosorp 250-4, 5µm, Merck®, Darmstadt) durchgeführt. Die chromatographischen Bedingungen waren:
- Proteinmenge pro Lauf:: 10 µg
- Flußgeschwindigkeit:: 0,5 mL * min⁻¹
- Detektionswellenlänge:: 280 nm
- Eluent A:: 0,1 % (v/v) Trifluoressigsäure (TFA) in H₂O
- Eluent B:: Acetonitril (> 99% v/v) + 0,1 % TFA

### 2.3.3. N-terminale Sequenzierung von PSA

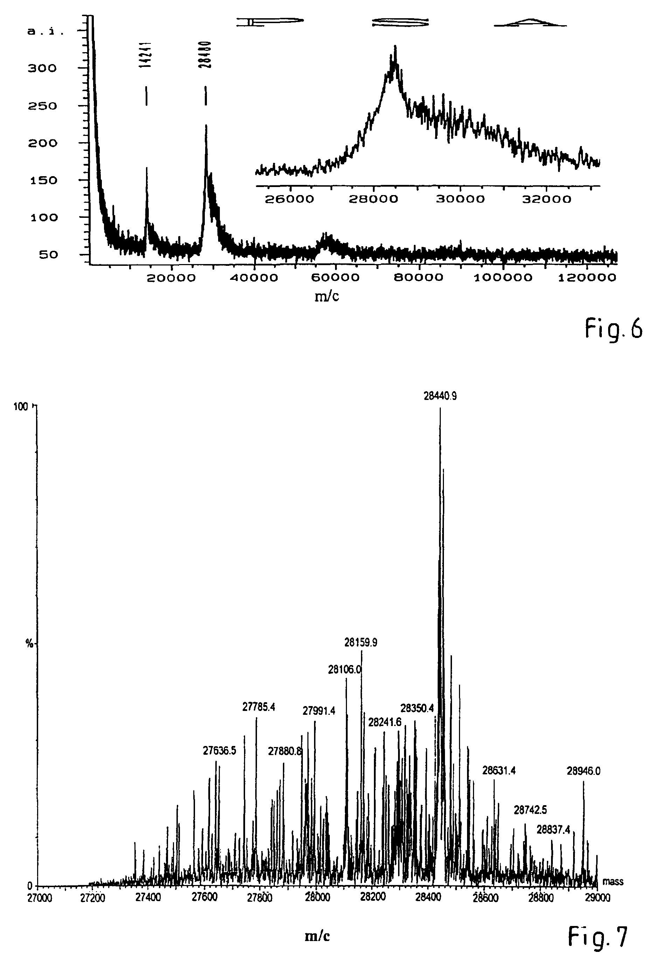

Mit einer Probe des gereinigten PSA wurde eine N-terminale Sequenzierung durchgeführt. Das Ergebnis ist in Fig. 4 gezeigt. Die Sequenzbereiche von PSA, die durch N-terminale Sequenzierung bestätigt wurden, sind in Fig.4 unterstrichen. Fett gedruckte Aminosäuren sind: N - Glykosilierungsstelle; KL - erste interne Schnittstelle; KS - zweite interne Schnittstelle. Die komplette Sequenz wurde aus der Datenbank MEDLINE erhalten. Die Bestimmung der N-terminalen Sequenz wurde mittels Edman-Abbau von membrangebundenem salzfreiem PSA erreicht. Es wurde ein Gasphasensequenzierer (Applied Biosystems®) strikt nach den Angaben des Herstellers verwendet.

Wie schon in der Literatur beschieben wurde (Watt et al, 1986 siehe die Beschreibungseinleitung) ist eine signifikante Menge von nativem PSA aus der Seminalflüssigkeit intramolekular gespalten. Aus diesem Grund wurden in der erfindungsgemäßen Substanz neben der Hauptsequenz beginnend mit IVGGWE... noch zwei weitere Sequenzen gefunden. Diese stammen von internen Schnittstellen nach Lysin 146 und Lysin 182. Da keine weiteren Aminosäuresequenzen innerhalb der ersten 10 Sequenzierungszyklen auftraten, kann die Reinheit der erfindungsgemäßen Substanz bezüglich des nativen PSA auf > 98% festgelegt werden, bedingt durch die Nachweisgrenze der verwendeten Methode.

Die erhaltenen Daten aus der Sequenzanalyse stimmen mit den Daten aus der SDS-PAGE überein: In Gegenwart des Reduktionsmittels β-Mercaptoethanol wurden neben der Bande bei ca. 33 kDa niedermolekulare Banden detektiert. Dies beruht auf der Tatsache, daß intern gespaltenes PSA nur noch durch reduzierbare Disulfidbrücken zusammengehalten wird. Aus diesem Grund sind ohne Reduktionsmittel die niedermolekularen Banden nicht sichtbar.

### 2.3.4. Hochauflösende HPLC

Die Trennung von intaktem und intern gespaltenem PSA war ebenfalls möglich mittels RP-18-HPLC bei Verwendung eines speziellen sehr flachen Gradienten und spezieller Säulen. Die Ergebnisse sind in Fig. 5 gezeigt, der die folgenden experimentellen Schritte zugrundeliegen: Eine hochauflösende HPLC wurde mit folgendem Gerät durchgeführt: (140A Solvent Delivery System, Perkin Elmer®, Applied Biosystems®); UV-detector bei 214 nm und Durchflußzelle von 2.4 µL -Länge 6 mm-(759A absorbance detector, Perkin Elmer®, Applied Biosystems®) und eine Injektonsschleife von 200 µL (7125, Rheodyne®). Die Chromatogramme wurden integriert (Modell D-2000 Chromato-Integrator, Merck-Hitachi®). Die Analysen wurden in allen Fällen bei einer konstanten Temperatur von +35 °C über eine Umkehrphasen C18-Säule durchgeführt (Dimensionen: I.D. 2.1 mm x 250 mm; Vydac® 218TP52). Der Gradient ist im Folgenden spezifiziert.

### Lösungen

- Eluent A:: 0.1 % (v/v) TFA in H₂O (HPLC-grade, Biosolve®);
- Eluent B:: 0.1 % (v/v) TFA in 70 % (v/v) Acetonitril (HPLC-grade, Biosolve®).

### Gradient

| | | | |
|---|---|---|---|
| Zeit | 0 min | 5 % (v/v) B | Fluß 80 µL/min |
| Zeit | 15 min | 47 % (v/v) B | Fluß 80 µL/min |
| Zeit | 160 min | 55 % (v/v) B | Fluß 80 µL/min |

Die manuell gesammelten Peaks A und B wurden einer Aminosäure-Sequenzanalyse unterworfen. Die N-terminale Sequenz des Materials unter Peak B entsprach genau der N-terminalen Sequenz von PSA ohne interne Schnittstelle (nur eine Sequenz wurde gefunden). Die Sequenzierung des Materials unter Peak A lieferte zwei Sequenzen nahezu gleicher Intensität: neben der regulären N-terminalen Sequenz wurde eine zweite gefunden, die den Aminosäuren 147 bis 155 von PSA entsprach. Wie bereits erwähnt, beruht die Spaltung zwischen den Aminosäuren 146 und 147 auf endoproteolytischer Aktivität des PSA (Watt et al, 1986 s.o.). Diese Spaltung tritt nicht während der Aufreinigung auf, sondern bereits im Seminalplasma, wie bereits eingangs bewiesen wurde.

### 2.3.5. Totalhydrolyse von PSA

Die Reinheit von PSA wurde zudem mittels totaler Hydrolyse bestimmt, wobei die Aminosäurezusammensetzung des erhaltenen PSA mit der berechneten Zusammensetzung verglichen wurde. Die Ergebnisse sind in Tabelle 4 aufgeführt, wobei die berechnete Zusammensetzung auf DNA-Sequenzanalysen basiert. Die Analyse der Aminosäurezusammensetzung von PSA wurde nach saurer Hydrolyse über die Phenyl-Thiohydantoin-Derivate mittels HPLC durchgeführt. Hierbei wurde ein automatisiertes System unter strikter Anwendung der Bedienungsvorschriften des Herstellers (Applied Biosystems®) verwendet. Die Derivatisierung fand mit Phenylisothiocyanat (PITC) statt.

**Tab. 4**

| **Vergleich der Aminosäurezusammensetzung von PSA mit der berechneten Zusammensetzung** | | | |
|---|---|---|---|
| Aminosäure (AS) | mol AS/mol PSA berechnet | mol AS/mol PSA gefunden | Verlust |
| A (Ala) | 12 | 12.0 | 0 |
| B (Asp + Asn) | 16 | 19.5 | -3.5 |
| C (Cys) | 10 | nicht nachweisbar | 10 |
| F (Phe) | 6 | 5.9 | 0.1 |
| G (Gly) | 20 | 18.3 | 1.7 |
| H (His) | 11 | 10.1 | 0.9 |
| I (Ile) | 8 | 5.8 | 2.2 |
| K (Lys) | 12 | 10.7 | 1.3 |
| L (Leu) | 25 | 23.7 | 1.3 |
| M (Met) | 4 | 2.3 | 1.7 |
| P (Pro) | 17 | 17.1 | -0.1 |
| R (Arg) | 10 | 9.7 | 0.3 |
| S (Ser) | 19 | 18.0 | 1 |
| T (Thr) | 13 | 13.8 | -0.8 |
| V (Val) | 22 | 17.3 | 4.7 |
| W (Trp) | 7 | nicht nachweisbar | 7 |
| Y (Tyr) | 4 | 3.8 | 0.2 |
| Z (Glx; Glu + Gln) | 21 | 22.1 | -1.1 |
| total | 237 | 210 | 27 |

Die Menge von 210 gefundenen Aminosäuren ist geringer als die berechnete Menge von 237. Dies beruht auf der Ausbeute des Systems (93 %) und der Tatsache, daß bei saurer Hydrolyse generell Cystein und Tryptophan zerstört werden. Aus diesem Grund kann auch die Totalhydrolyse als weiteres Reinheitskriterium der erfindungsgemäßen Substanz gewertet werden. Zudem zeigen Referenzproteine wie Myoglobin ähnliche Abweichungen wie dies bei PSA der Fall war.

### 2.3.6. Massenspektroskopische Analysen von PSA

Eine Bestimmung der Masse von Proteinen erfolgt in den meisten Fällen mittels SDS-PAGE. Hierbei wurde im Stand der Technik gefunden, daß PSA anscheinend eine Masse von 33 kDa besitzt. Neuerdings können wesentlich genauere Bestimmungen mittels Massenspektroskopie durchgeführt werden.

Mittels MALDI (matrix assisted laser desorption ionisation) Massenspektroskopie, deren Erbegnis in Fig. 6 gezeigt ist, wurde gefunden, daß die Hauptmasse von PSA bei m/c = 28480 Da liegt. Offensichtlich ist das erhaltene PSA ein heterogenes Gemisch von Spezies unterschiedlicher Massen zwischen ca. 26 und 34 kDa. Weitere Massenpeaks entsprechen unterschiedlich geladenen Addukten (m/2c und 2m/c).

Fig. 6 liegen die folgenden experimentellen Schritte zugrunde: Die Massenspektroskopie wurde mittels MALDI nach üblicher Vorschrift durchgeführt. Für jede Messung wurde salzfreies lyophilisiertes Protein verwendet, welches mit H₂O zu einer Lösung von ca. 10 pmol µL⁻¹ rekonstituiert wurde. Die MALDI-Massenspektren wurden mit einem Bruker® Reflex III Instrument (Bruker-Franzen Analytik GmbH®) aufgenommen. Die Ionisierung erfolgte mit einem Stickstofflaser (337 nm, 3 ns Pulsbreite, 3Hz). Spektren wurdem im Linearmodus erhalten. Es fand eine externe Kalibrierung mit Pferdeherz-Myoglobin statt (Sigma Kat. Nr. M1882-minimum 90 %, lyophilisiert, salzfrei). Die Kalibrierlösung von Pferdeherz-Myoglobin wurde eingestellt auf eine Konzentration von 1,5 pmol/µL in 0,1 % (v/v) Essigsäure in Acetonitril und Wasser in einem Mischungsverhältnis von 50 : 50 (v/v). Die durchschnittliche molekulare Masse von Pferdeherz-Myoglobin beträgt 16951,48 (nach IUPAC). Unterschiedlich geladene Molekülspezies dienten der Kalibrierung des Systems.

Die entsprechenden m/c-Werte lagen bei 808,221; 848,582; 893,191; 942,757; 998,154; 1060,475; 1131,107; 1211,828; 1304,968; 1413,631; 1542,051; 1696,156; 1884,506; 2119,943. Als Matrix für die Messungen diente α-Cyano-4-Hydroxyzimtsäure in Octyl-Glucosid. Pro Messung wurden ca. 1 pmol Probe in die Matrix eingebracht und mit 2 µL 0.1 % (v/v) TFA gewaschen. Es wurde danach die sogenannte Dünnschichtmethode angewandt. Spektren wurden aus 80 Einzelionisierungen zusammengesetzt, mit einer 52-fachen Laser-Verstärkung. Alle weiteren Vorgänge wurden strikt nach der Bedienungsanleitung des Herstellers durchgeführt.

Wie aus dem Massenspektrum ersichtlich ist, zeigt PSA eine Massenverteilung von ca. 26 bis 34 kDa, wobei oberhalb von 32 kD nur noch vereinzelte Isoformen des PSA in geringer Konzentration auftreten. Die generelle Massenverteilung beruht offensichtlich auf unterschiedlichen Seitenketten glykosidischer Natur, da der Proteinteil immer gleich ist, wie weiter oben bewiesen wurde. Es ist somit auch gezeigt worden, daß eine Massenabschätzung mittels SDS-PAGE nur dann einigermaßen korrekte Daten liefert, wenn kein Reduktionsmittel zugesetzt wird. Eine mögliche Erklärung könnte darin liegen, daß PSA ein Gemisch unterschiedlich glykosilierter Isoformen ist. Bei solchen Molekülen ist bekannt, daß sie keinen "Random Coil" in Gegenwart von SDS bilden. Da sich aber nur bei"Random Coils" die Wanderungsstrecke und das Molekulargewicht reziprok verhalten, zeigt die SDS-PAGE zu hohe Massen an.

Neben MALDI kann eine exakte Bestimmung der Molekülmasse mittels Elektrospray-Ionisierung und anschließender Massenspektroskopie erfolgen. Ein typisches Massensprektrum nach Elektrospray-Ionisierung ist in Fig. 7 gezeigt, wobei Fig. 7 die folgenden experimentellen Schritte zugrundeliegen: Die Elektrospray-Ionisierung fand im positiven Modus statt. Spektren wurden mit einem VG-Plattform-Massenspektrometer (Fisons®) aufgenommen, verbunden mit einer Elektronenquelle unter atmosphärischem Druck und einem Quadrupol-Massenspektrometer. Die Auflösung wurde auf ca. 1000 gesetzt. Das Gerät wurde nach den Angaben des Herstellers gegen Myoglobin kalibriert und getunt. Es fand eine externe Kalibrierung mit Pferdeherz-Myoglobin statt (Sigma Kat. Nr. M1882-minimum 90 %, lyophilisiert, salzfrei). Die Kalibrierlösung von Pferdeherz-Myoglobin wurde eingestellt auf eine Konzentration von 15 pmol/µL in 0,1 % (v/v) Essigsäure in Acetonitril und Wasser in einem Mischungsverhältnis von 50 : 50 (v/v). Die Kalibrierlösung wurde über einen Injektor (10 µL Schleife) mit einer Flußrate von 5 µL/min injiziert. Die durchschnittliche molekulare Masse von Pferdeherz-Myoglobin beträgt 16951,48 (nach IUPAC). Unterschiedlich geladene Molekülspezies dienten der Kalibrierung des Systems. Die entsprechenden m/c-Werte lagen bei 808,221; 848,582; 893,191; 942,757; 998,154; 1060,475; 1131,107; 1211,828; 1304,968; 1413,631; 1542,051; 1696,156; 1884,506; 2119,943. Generell wurden Spektren im Bereich von 800 bis zu 34000 Da aufgenommen mit einer Scan-Rate von 7 s/Scan. PSA-Proben wurden in 20% (v/v) Acetonitril in Wasser + 0.1 % (v/v) Trifluoressigsäure aufgenommen und auf eine Konzentration von ca. 15 pmol/µL eingestellt. Die Proben wurden mittels eines Injektors (10 µL Schleife) bei einer Flußrate von 5 µL/min in die Elektrospray-Ionisationskammer eingeführt.

### 2.3.7. Bestimmung des Kohlenhydrat-Anteils von PSA

Der kalkulierte isoelektrische Punkt des reinen Proteinteils von PSA liegt bei pI = 7,2. Vergleicht man diesen Wert mit den tatsächlichen isoelektrischen Punkten des PSA aus der vorliegenden Präparation stellt man fest, daß alle PSA-Isoformen niedrigere isoelektrische Punkte aufweisen. Somit müssen bei allen Isoformen zusätzliche saure Komponenten angeheftet sein. Hierbei kann es sich natürlicherweise entweder um Glycoside oder um Phosphatgruppen handeln. Da normalerweise in humanen glycosidischen Seitenketten ein hoher Anteil saurer Zucker vorliegt, bestand die erste Hypothese darin, daß das vorliegende PSA glycosiliert ist.

Um diese Hypothese zu beweisen, wurde eine entsalzte Probe (ca. 0,1 mg) einer Methanolyse unterworfen. Hierbei wurde die wasserfreie Probe in 1 mol/L HCl in wasserfreiem Methanol 16 h auf 80 °C erhitzt. Die erhaltenen Methyl-Gycoside wurden mit Essigsäureanhydrid N-acetyliert. Anschließend wurden die Proben noch trimethyl-silyliert und mittels Gaschromatographie analysiert. Als Säule wurde eine Supelco® DB-1 fused Silica Kapillarsäule einer Länge von 30 m verwendet. Der Temperaturgradient betrug 2 °C/min (von 80 °C Initialtemperatur bis zu 160 °C Endtemperatur). Die Einzelkomponenten wurden mittels Ko-Chromatographie mit Referenzsubstanzen identifiziert. Dabei wurden folgende Ergebnisse erhalten:

| Kohlenhydrat | Masse / % |
|---|---|
| Mannose | 6.6 |
| Galactose | 2.4 |
| Glucose | 1.0 |
| N-Acetylglucosamin | 6.9 |
| Inositol | 2.7 |
| Acetyl-Neuraminsäure | 3.5 |

Um festzustellen, welchen Massenanteil die glycosidischen Seitenketten an der PSA-Gesamtmasse ausmachen, wurden ca. 100 µg salzfreies PSA mit N-Glycanase (Genzyme®, Boston, MA; 1.0 U) in 0,25 mL einer Lösung von 0,1 mol/L NH₄HCO₃ bei 37 °C für 12 h inkubiert. Anschließend wurde diese Lösung auf eine RP-C18-Säule aufgetragen und das Material, welches im Ausschlußvolumen eluierte, vereinigt und lyophilisiert. Dieses lyophilisierte Material wurde mittels MALDI-MS analysiert. Hierbei zeigte sich im MALDI-Massenspektrum ein Peak bei ca. 2980 Da. Diese Masse wurde in Verhältnis gesetzt zur Masse intaktem PSA von 28480 Da und somit ein Carbohydratanteil der gesamten Masse von ca. 10,5 % berechnet.

### 2.3.8. Nachweis von Phosphat-Resten in der vorliegenden PSA-Präparation

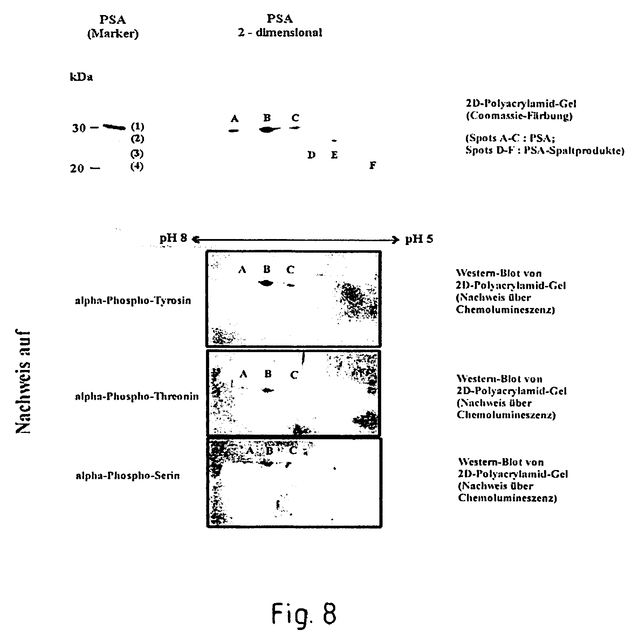

Wie bereits im vorhergehenden Abschnitt erwähnt, können neben Glycosiden auch Phosphatreste natürlicherweise zu einer Absenkung des isoelektrischen Punktes führen. Diese generelle Hypothese wurde für PSA mittels Westernblot geprüft. Hierbei zeigte sich erstaunlicherweise, daß offensichtlich in der vorliegenden PSA-Präparation Phospho-Tyrosin vorliegt. Mit wesentlich geringerer Intensität wurden im Westernblot auch Phospho-Threonin und Phospho-Serin detektiert, was in Fig. 8 gezeigt ist, der die folgenden experimentellen Schritte zugrundeliegen: Die erfindungsgemäße PSA-haltige Substanz wurde unter reduzierenden Bedingungen mittels zweidimensional-gekoppleter PAGE und IEF in seine Isoformen aufgetrennt. Anschließend wurde auf Nitrozellulose geblottet und ein Westernblot mit anti-α-Phosphotyrosin; anti-α-Phosphothreonin und anti-α-Phosphoserin durchgeführt. Es fand in allen Fällen eine Detektion mittels Chemoluminiszenz statt.

Der Nachweis von phosphoriliertem PSA ist überraschend, da in der gesamten Literatur kein Hinweis auf Phosphorilierung dieses Tumormarkers vorliegt. Dennoch würde die Phosphorilierung zum ersten Mal erklären, warum im Serum trotz großen Überschusses an PSA-Komplexbildnnern ( z.B. ACT) ein signifikanter Anteil nicht gebunden wird und als freies PSA vorliegt.

### 3. Isoformspezifischer Nachweis von PSA

Wie bereits in der Beschreibungseinleitung angedeutet, besteht die Möglichkeit, daß bestimmte Isoformen präferenziell von Tumorgewebe gebildet und exkretiert werden können. Somit könnte ein isoformspezifischer Nachweis von PSA die diagnostische Spezifität wesentlich erhöhen. Da im Stand der Technik weder isoformspezifische Nachweissysteme publiziert sind und auch keine isoformspezifischen Antikörper verfügbar sind, bestand zunächst die Aufgabe darin, isoformspezifische monoklonale Antikörper zu gewinnen. Mit solchen Antikörpern können dann isoformspezifische immunologische Testsysteme entwickelt werden.

### 3.1. Methoden

### 3.1.1. Immunisierung

Immunisiert wurden drei Balb/c Mäuse mit je 20 µg der erfindungsgeäßen PSA-haltigen Substanz aufgenommen in 200 µL PBS/komplettem Freundschen Adjuvanz (1:1) am Tag 18. vor der Fusion. Die 2. Immunisierung erfolgte 3 Tage später (Tag 15. vor der Fusion) mit 20 µg PSA in 200 µL PBS / in komplettem Freundschen Adjuvanz (1:1) je Maus. Nachfolgend wurden am Tag 11, 8, 5 und 1 vor der Fusion je Maus 20 µg PSA in 200 µL PBS injiziert. Die Immunisierungen erfolgten durch lokale subkutane Injektion in der Nähe des poplitealen Lymphknotens.

### 3.1.2. Lymphocytenpräparation

Nach Töten der Maus wurden die poplitealen Lymphknoten freigelegt, herauspräpariert und in warmes Medium gegeben (Optimen, 5% FCS, 1% Glutamin, 1% Penizillin/Strepdavidin). Zur Gewinnung der Lymphozyten wurde der Lymphknoten angeschnitten und zwischen zwei Objektträgern zerrieben. Das Medium mit den Lymphozyten wurde zentrifugiert (5 min, 950 upm), der Überstand verworfen und die abzentrifugierten Lymphozyten in die Fusion eingesetzt.

### 3.1.3. Zellfusion

Für die Zellfusion wurden die gewonnenen Lymphozyten und die Myelom-Zellen (AG8) in HEPES-Medium suspendiert. Die vereinigten Zellen (Lymphozyten und Myelom-Zellen) wurden aufkonzentriert (Zentrifugation: 5 min, 950 upm) mit 1 mL PEG-Lösung versetzt (PEG 4000, 5g auf 5 mL HEPES-Medium), 2 min bei 37°C geschüttelt und nach Zugabe von 20 mL HEPES-Medium 5 min bei 37°C inkubiert. Nach dem Wechsel auf HA-Medium wurde die Zellsuspension auf 15 24-Well-Platten verteilt und bei 37°C im Brutschrank inkubiert. Nach 4 Tagen wurden die Zellen mit 1 mL HA-Medium pro Well gefüttert. Nach 10 Tagen wurden die Überstände zum Testen abgenommen. Positiv getestete Zellen wurden mit DMSO Zusatz nach Standardprotokoll eingefroren.

### 3.1.4. Screening nach positiven Klonen

Das Screening nach anti-PSA-Antikörper produzierenden Klonen erfolgte im ELISA. Hierzu wurden PSA beschichtete und mit 10% FCS abgesättigte Mikrotiterplatten mit je 100 µL Kulturüberstand pro Well versetzt. Als negative Kontrolle wurde nur Medium, als positive Kontrolle Medium mit einem schon verfügbaren anti-PSA-Antikörper (PSA 43A2) benutzt. Alle Bestimmungen wurden als Doppelbestimmung ausgeführt. Nach 1 h Inkubation bei 37°C wurden die Platten 3x mit PBS gewaschen und pro Weil der 2. Antikörper (Ziege anti-Maus-IgG, peroxidasegekoppelt) zugesetzt. Überschüssiger 2. Antikörper wurde nach 1 h Inkubation bei 37°C durch 3x Waschen mit PBS entfernt. Die proportional zur anti-PSA-Antikörpermenge ablaufende Farbreaktion wurde durch Zugabe des Substratpuffers (50 mL 0,05 mol/L Na₂HPO₄, 0,025 mol/L Citronensäure; 20 mg 1,2-Phenylendiamin; 50 µL H₂O₂) gestartet. Nach deutlicher Farbentwicklung in der positiven Kontrolle wurde die Reaktion durch Zugabe von 2,5 mol/L H₂SO₄ abgestoppt und im ELISA-Reader (SLT) ausgewertet.

### 3.1.5. Subklassenbestimmung

Für den Subklassentest wurden Mikrotiterplatten mit Ziege-anti-Maus Antikörper beschichtet (Puffer: 100 mol/L Carbonatpuffer, pH 9,0; Inkubation: 16 h, 4°C). Die Absättigung der Platten erfolgte mit 10% FCS / PBS (Inkubation: 1 h, 37°C). Der zu testende Antikörper wurde als Kulturüberstand auf 12 Wells verteilt und 1 h bei 37°C inkubiert. Die Bestimmung der Subklasse erfolgte durch Peroxidase gekoppelte subklassenspezifische Antikörper in Doppelbestimmung (IgG1, IgG2a, IgG2b, IgG3 spezifische Antikörper (caltec); IgM spezifischer Antikörper (medac); IgG+IgM spezifischer Antikörper (Dianova); Inkubation: 1 h bei 37°C). Als Substrat wurde ortho-Phenylendiamin eingesetzt und die Farbreaktion mit 2,5 mol/L Schwefelsäure gestoppt.

### 3.2. Ergebnisse

Bei der Darstellung monoklonaler anti-PSA Antikörper zeigten 67 Klone im PSA-ELISA eine positive Reaktion. Hiervon wurden 33 Klone mit starken Signalen im ELISA als Einzelklone gehalten und die übrigen in 7 Pools zusammengeführt. Im Subklassentest erwiesen sich 11 Klone vom Typ IgM, 12 Klone vom Typ IgG1 und die restlichen 10 als nicht reine Klone mit den Subklassen IgM und IgG1 (Tab. 3).

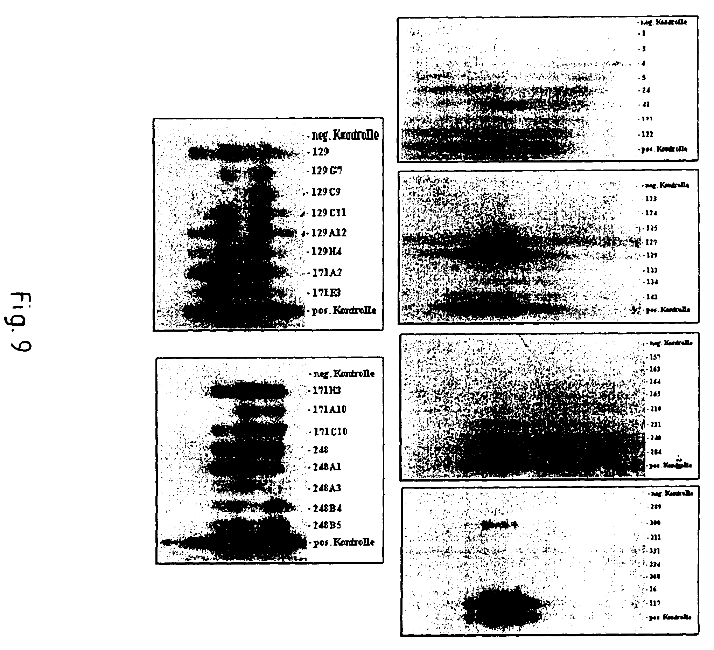

Die Antikörper wurden anschließend auf ihre Spezifität gegenüber einzelnen Banden im IEF-Gel überprüft. Hierzu wurde das im IEF-Gel aufgetrennte PSA im Westernblot-Verfahren auf eine Immobilon Membran übertragen. Die einzelnen Antikörper wurden danach in der Schlitzkammerapparatur geprüft. Hierbei zeigte sich, daß 8 der 33 Klone Antikörper produzieren, die mit einzelnen Banden reagieren. Von den 8 Klonen wurden 3 subkloniert und ergaben 14 Subklone. Hierdurch ließen sich einzelne Subklone gewinnen, die eine höhere spezifität gegenüber einzelnen Banden des IEF-Westernblots aufwiesen als der Ausgangsklon, wie dies auch aus Tabelle 5 und Fig. 9 hervorgeht. Dabei liegen Fig. 9 die folgenden experimentellen Schritte zugrunde: Als Marker wurde Proteinstandard (Gibco BRL, 10 kDa Leiter) verwendet; zur Prüfung auf unspezifische Reaktionen wurde als negative Kontrolle fötales Kälberserum (FCS) eingesetzt; die immunologische Reaktion wurde mit einem monoklonalen PSA spezifischen Antikörper (43A2; SERATEC) durchgeführt.

**Tabelle 5**

| **Listung der im ELISA positiven Antikörper** | | | |
|---|---|---|---|
| Ursprungsklon | Subklon | Subklasse | IEF pH 5-8 -Westernblot |
| 1 | | IgM, IgG1 | - |
| 3 | | IgM, IgG1 | - |
| 16 | | IgM | Bande: pH 6,0 |
| 24 | | IgG1 | - |
| 41 | | IgG1 | Banden: pH 6,0 + 6,8 |
| 117 | | IgG1 | alle Banden |
| 127 | | IgM | Bande: pH 6,0 |
| 129 | A12 | IgG1 | Banden: pH 6,0 + 6,8 + 7,2 |
| | C9 | IgG1 | Bande: pH 6,0 |
| | C11 | IgG1 | Banden: pH 6,0 + 6,8 |
| | G7 | IgG1 | Banden: pH 6,0 + 6,8 |
| | H4 | IgG1 | Banden: pH 6,0 + 6,4 + 6,8 |
| 171 | A2 | IgG1 | Banden: pH 6,0 + 6,4 + 6,8 |
| | A10 | IgG1 | Banden: pH 6,0 + 6,4 + 6,8 |
| | C10 | IgG1 | Banden: pH 6,0 + 6,4 + 6,8 + 7,2 |
| | E3 | IgG1 | Banden: pH 6,0 + 6,4 + 6,8 |
| | H3 | IgG1 | Banden: pH 6,0 + 6,4 + 6,8 + 7,2 |
| 248 | A1 | IgG1 | Banden: pH 6,4 + 6,8 + 7,2 |
| | A3 | IgG1 | Bande: pH 6,8 |
| | B4 | IgG1 | Banden: pH 6,0 + 6,8 |
| | B5 | IgG1 | Banden: pH 6,0 + 6,4 + 6,8 |
| 300 | | IgM, IgG1 | Banden: pH 6,0 + 6,4 + 6,8 |

## Patentansprüche

1. Substanz, deren Gesamtkonzentration an Prostata-spezifischem Antigen (PSA) mit einer N-terminalen Aminosäuresequenz der Folge IVGGWECEKHSQPWQVLVAS mindestens 90 Gewichts-% beträgt, wobei mindestens eine Isoform des PSA, deren isoelektrischer Punkt um mindestens 0,6 pI unter dem isoelektrischen Punkt von nicht-glykosiliertem PSA liegt, in einem Anteil von mindestens 3,0 % der PSA-Gesamtkonzentration vorliegt.

2. Substanz nach Anspruch 1, **dadurch gekennzeichnet**, daß mindestens eine Isoform des PSA, deren isoelektrischer Punkt um mehr als 1,0 pI unter dem isoelektrischen Punkt von nichtglykosiliertem PSA liegt, in einem Anteil von mindestens 3,0 % der PSA-Gesamtkonzentration vorliegt.

3. Substanz nach Anspruch 1 oder 2, **dadurch gekennzeichnet**, daß mindestens vier Isoformen des PSA, die unterschiedliche isoelektrische Punkte aufweisen, in einem Anteil von jeweils mindestens 3,0 % der PSA-Gesamtkonzentration vorliegen.

4. Substanz nach Anspruch 3, **dadurch gekennzeichnet**, daß fünf Isoformen des PSA, die unterschiedliche isoelektrische Punkte aufweisen, in einem Anteil von jeweils mindestens 3,0 % der PSA-Gesamtkonzentration vorliegen.

5. Substanz nach Anspruch 4, **dadurch gekennzeichnet**, daß eine erste Isoform des PSA einen isoelektrischen Punkt von 5,6 bis 5,8 aufweist und in einem Anteil von 3 bis 5 % der PSA-Gesamtkonzentration vorliegt, daß eine zweite Isoform des PSA einen isoelektrischen Punkt von 6,1 bis 6,4 aufweist und in einem Anteil von 9 bis 13 % der PSA-Gesamtkonzentration vorliegt, daß eine dritte Isoform des PSA einen isoelektrischen Punkt von 6,6 bis 6,8 aufweist und in einem Anteil von 34 bis 42 % der PSA-Gesamtkonzentration vorliegt, daß eine vierte Isoform des PSA einen isoelektrischen Punkt von 6,9 bis 7,1 aufweist und in einem Anteil von 25 bis 31 % der PSA-Gesamtkonzentration vorliegt und daß eine fünfte Isoform des PSA einen isoelektrischen Punkt von 7,1 bis 7,3 aufweist und in einem Anteil von 10 bis 14 % der PSA-Gesamtkonzentration vorliegt.

6. Substanz nach einem der Ansprüche 1 bis 5, **dadurch gekennzeichnet**, daß die PSA-Gesamtkonzentration mindestens 95 Gewichts-%, vorzugsweise mindestens 98 Gewichts-% beträgt.

7. Substanz nach einem der Ansprüche 1 bis 6, **dadurch gekennzeichnet**, daß die molekularen Massen der Isoformen des PSA mit dem Anteil von jeweils mindestens 3,0 % der PSA-Gesamtkonzentration zwischen 26000 und 32000 Da liegen.

8. Substanz nach einem der Ansprüche 1 bis 7, **dadurch gekennzeichnet**, daß mindestens 3,0 %, vorzugsweise mindestens 10 % der PSA-Gesamtkonzentration phosphoriliert sind, wobei der phosphorilierte Anteil der PSA-Gesamtkonzentration bestimmt ist.

9. Substanz nach Anspruch 8, **dadurch gekennzeichnet**, daß mindestens eine Isoform des PSA, deren isoelektrischer Punkt um mindestens 0,4 pI unter dem isoelektrischen Punkt von nicht-glykosiliertem PSA liegt und die den Anteil von mindestens 3,0 % der PSA-Gesamtkonzentration aufweist, zu mindestens 50 % phosphoriliert ist.

10. Verwendung einer Substanz nach einem der Ansprüche 1 bis 9 als Referenzsubstanz bei der Bestimmung der Konzentration von PSA in menschlichem Blut oder Serum.

11. Verwendung einer Substanz nach einem der Ansprüche 1 bis 9 bei der Erzeugung von monoklonalen isoformspezifischen PSA-Antikörpern.
